# EUROPÄISCHE PATENTSCHRIFT

(11) **EP 0 783 870 B1**
(45) Veröffentlichungstag und Bekanntmachung des Hinweises auf die Patenterteilung: **28.03.2001**
(21) Anmeldenummer: 96119147.5
(22) Anmeldetag: 29.11.1996
(51) Int. Cl.: A61B 8/08, A61B 17/225

(54) **Vorrichtung zur Ortung von Konkrementen im Körper eines Patienten**
Device for locating concretions in the body of a patient
Dispositif pour la localisation de concrétions dans le corps d'un patient

(30) Priorität: 21.12.1995 DE 19548000
(43) Veröffentlichungstag der Anmeldung: 16.07.1997
(73) Patentinhaber: Dornier MedTech Holding International GmbH, 82234 Wessling (DE)
(72) Erfinder: Überle, Friedrich, Dipl.-Ing., Dr., 82205 Gilching (DE)

(56) Entgegenhaltungen:
- EP-A- 0 388 636
- DE-A- 4 212 809
- US-A- 5 072 733

## Beschreibung

Die Erfindung betrifft eine Vorrichtung zur Ortung von Konkrementen im Körper eines Patienten, bestehend aus einer Schallquelle, einer Koppelanordnung zwischen Schallquelle und Patient zur Einleitung der erzeugten Schallwellen in den Körper des Patienten und aus einer Anordnung zur Erzeugung einer Markierung auf der Haut des Patienten.

Anordnungen zur Erzeugung von Markierungen auf der Hautoberfläche eines zu therapierenden Patienten sind bekannt und üblicherweise speziell auf die zugehörige Ortungsvorrichtung abgestimmt, mit der sie mechanisch und/oder elektrisch verbunden sind. Mit einer derartigen Anordnung zur Markierung ist es möglich, nach Auffinden des zu zertrümmernden Konkrementes mittels der Ortungsvorrichtung die Hautoberfläche z.B. optisch zu markieren, so daß der Therapiekopf, welcher die Stoßwellen zur Zertrümmerung des Konkrementes erzeugt, in einem vorgegebenen Abstand auf diese Markierung ausgerichtet wird, um so ein optimales Einleiten der Therapiestoßwellen in den Körper zu ermöglichen.

So beschreibt die DE-A-40 03 350 der Anmelderin eine Vorrichtung zum Justieren eines zustellbaren C-Bogen-Röntgengeräts, mit Röntgenröhre und Bildverstärker an eine Vorrichtung zum Behandeln von Patienten mit extrakorporal erzeugten fokussierten Stoßwellen, insbesondere an einem Lithotripter, wobei mindestens zwei am Röntgengerät befestigte Lichtstrahlen erzeugende Apparaturen, deren Strahlen sich im Isozentrum des C-Bogens kreuzen, und ein an der Therapieeinheit des Stoßwellengeräts anbringbares Referenzelement, das die Lage des Therapiefokus anzeigt, vorgesehen sind.

Des weiteren beschreibt die DE-A-40 33 439 der Anmelderin eine Zieleinrichtung zur Röntgenortung bei einer extrakorporalen Behandlung von Patienten mit fokussierten Stoßwellen, insbesondere für einen Lithotripter, wobei die Zieleinrichtung aus einem Träger besteht auf oder in dem röntgenpositive Elemente so angeordnet sind, daß sie auf den Brennpunkt des Stoßwellensystems weisen, wobei die lage der Elemente im Röntgenbild eindeutig die Lage des Brennpunkts erkennen läßt.

Bei beiden bekannten Einrichtungen sind die jeweiligen Markierungsanordnungen, wie eingangs erwähnt, speziell auf die zugehörigen Ortungsanordnungen ausgerichtet und lassen sich nicht mit anderen Lithotriptern bzw. deren Ortungsanordnungen einsetzen.

Aufgabe der vorliegenden Erfindung ist es, eine Anordnung zur Erzeugung einer Markierung eines Patienten zu schaffen, die mit einer beliebigen Schallquelle zusammenarbeitet und die es ermöglicht, an einer frei wählbaren Stelle des Schallbildes die Markierung einzublenden, so daß der Fokus für das Schalltherapiegerät markiert werden kann.

Ausgehend von einer Vorrichtung der eingangs näher genannten Art erfolgt die Lösung dieser Aufgabe mit den im kennzeichnenden Teil des Anspruchs 1 angegebenen Merkmalen; vorteilhafte Ausgestaltungen sind in den Unteransprüchen beschrieben.

Mit der erfindungsgemäßen Vorrichtung ist es also möglich, eine geeignete Markierung, z.B. ein Fadenkreuz oder einen Lichtpunkt, an einer beliebigen Stelle in dem Schallbild einzublenden, und zwar ohne elektrischen Eingriff in die Steuerungsanordnung der Schallquelle, wobei diese beliebig ausgestaltet sein kann.

Im folgenden wird die Erfindung anhand der Zeichnung näher erläutert, in der vorteilhafte Ausführungsformen dargestellt sind. Es zeigen:
Fig. 1 einen schematischen Schnitt durch einen Teil der Ortungsanordnung und des Patienten;
Fig. 2 einen Schnitt durch einen Teil der Ortungsanordnung und des Therapiekopfes;
Fig. 3 eine in ein Schallbild eingeblendete Markierung und
Fig. 4 ein Diagramm einer möglichen geeigneten Impulsfolge.

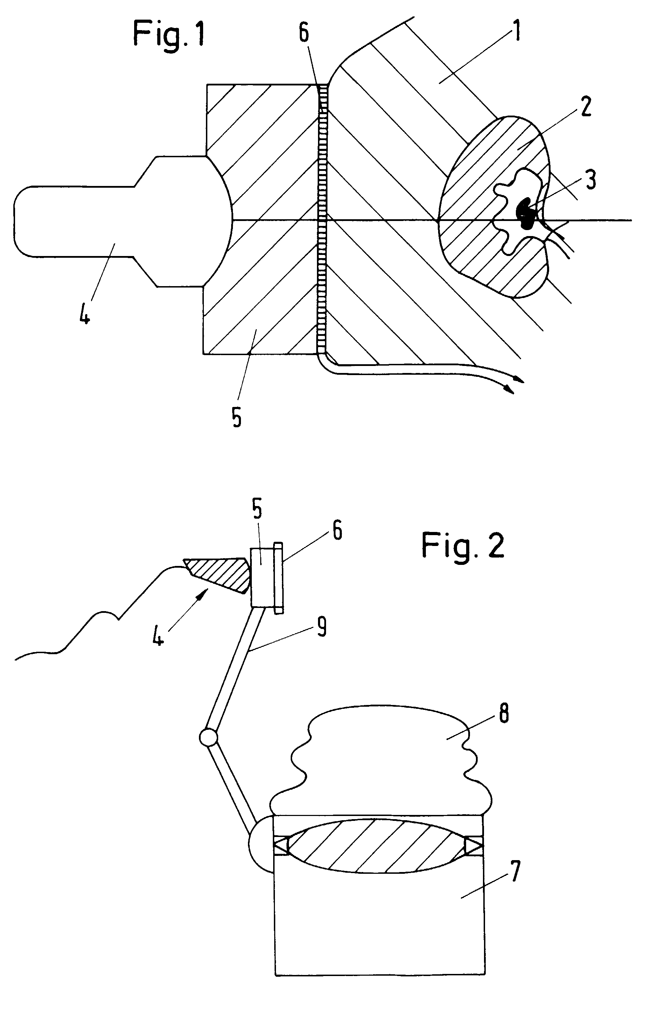

In Fig. 1 ist mit 1 ein Teil eines Körpers eines Patienten schematisch dargestellt, in dem ein Konkrement, z.B. ein Stein 3 in einer Niere 2, durch die von einem in dieser Figur nicht dargestellten Therapiekopf ausgesandten Stoßwellen zertrümmert werden soll. Um nun die genaue Lage des Konkrementes 3 im Körper des Patienten 1 zu orten, ist eine Schallquelle 4 in herkömmlicher Weise vorgesehen, die über eine Koppelanordnung 5 zum Patienten ausgerichtet wird, so daß nach Auffinden des Konkrementes 3 durch in unterschiedliche Richtungen ausgesandte Schallwellen auf einer (nicht dargestellten) Anordnung ein Schallbild des Konkrementes erzeugt werden kann.

Erfindungsgemäß ist nun vorgesehen, daß der Schallquelle 4 ein Sensor 6 zugeordnet ist, der für die verwendeten Schallwellen transparent ist und der eine Markierung erzeugt, welche in vorgegebener Weise z.B. als Lichtpunkt oder Fadenkreuz in das vom Konkrement 3 erhaltene Schallbild eingeblendet wird.

Zu diesem Zweck besteht der Sensor 6, dessen Abmessung vorteilhafterweise dem Durchmesser des Senders (Scanners) der ausgesandten Schallwellen entspricht, aus einer Vielzahl von einzelnen Zellen, die so ausgestaltet sind, daß nach einer z.B. elektrisch einstellbaren Zeit, die der Laufzeit des Schalls zum Konkrement und zurück entspricht, ein Signal erzeugt wird, welches von der Anordnung zur Sichtbarmachung des Schallbildes als Reflex aus einer vorgegebenen Tiefe abgebildet wird. Dieses Bild kann dabei ein Lichtpunkt, ein Fadenkreuz oder eine anders ausgestaltete Markierung sein, die an einer beliebigen Stelle ohne elektrischen Eingriff in die elektrische Versorgung der Schallquelle in das Schallbild eingeblendet werden kann.

Es ist klar, daß damit beliebige Schall-Scanner im Zusammenhang mit dem erfindungsgemäßen Sensor zur Erzeugung einer Markierung verwendet werden können, da jegliche Art der speziellen Anpassung der Markierungsanordnung an die verwendete Schallquelle entfällt.

Fig. 2 zeigt eine Anordnung, bei der der Sensor 6 mit der zugehörigen Koppelanordnung an einer Halterung befestigt ist, die wiederum mit einem Therapiekopf 7 eines Stoßwellengenerators verbunden ist, welcher über eine eigene Koppelanordnung 8 mit dem Körper des Patienten in Verbindung steht. Die Halterung 9 ermöglicht eine feste Zuordnung des Sensors 6 zum Therapiekopf 7. Auch hier ist der Sensor 6 wieder derart ausgestaltet, daß nach dem Auftreffen des von der Schallquelle 4 ausgesandten Ortungs-Schallimpulses und nach Ablauf einer vorgegebenen Verzögerungszeit ein Signal vom Sensor 6 ausgesandt wird, welches der doppelten Laufzeit des Schalls zur geforderten Entfernung im Körper des Patienten entspricht, so daß dieses Signal von der Anordnung zur Darstellung des Schallbildes wie ein Reflex aus dieser vorgegebenen Tiefe abgebildet wird.

Durch entsprechende Auslegung der Oberfläche und des Aufbaus des Sensors 6 als Array bzw. mechanische Ausformung ist eine beliebige Art der Markierung erzielbar, wie z.B. ein Fadenkreuz, ein Punkt oder ein Kreis.

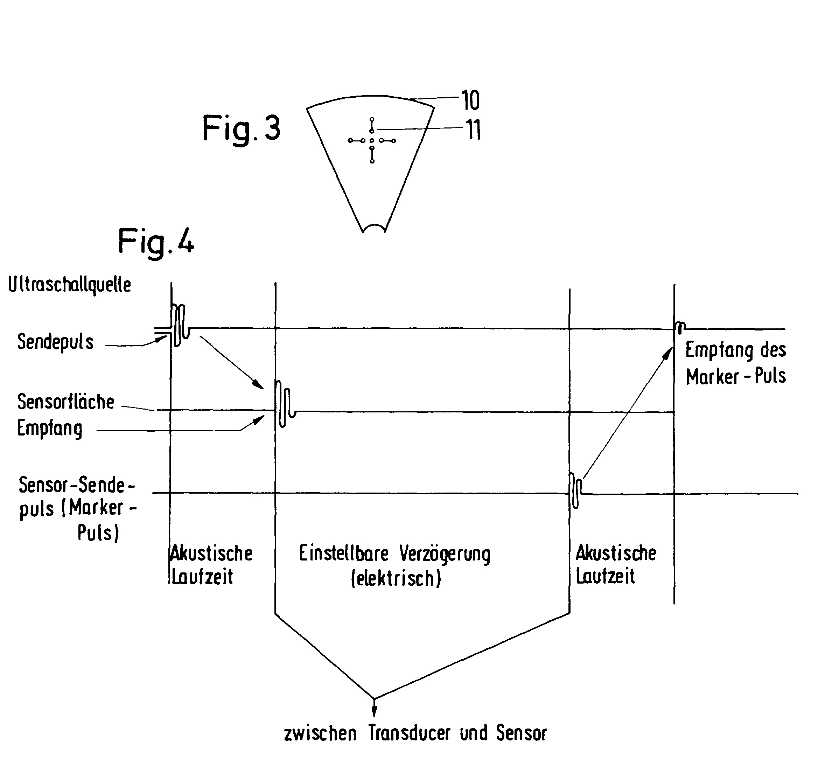

Fig. 3 zeigt schematisch das von der Anordnung zur Darstellung des Schalles erzeugte Schallbild 10, mit einem z.B. akustisch eingeblendeten Fadenkreuz 11, welches vom Sensor 6 erzeugt worden ist.

Fig. 4 zeigt ein mögliches Diagramm einer Impulsfolge, aus dem hervorgeht, daß nach dem Auftreffen des von der Schallquelle der Ortungsanordnung ausgesandten Sendepulses auf die Sensorfläche und nach Ablauf einer z.B. elektrisch einstellbaren Verzögerungszeit, der Sensor ein Ausgangssignal erzeugt, welches als Markierungspuls im Schallbild 10 dargestellt wird.

Diese vom Sensor abgegebenen Signale können ortsselektiv ausgesandt werden, d.h. sie werden nur dann erzeugt, wenn eine oder mehrere bestimmte Abtastlinien detektiert werden; sie können aber auch in ihrer Form bzw. in ihrem Frequenzgehalt derart modifiziert werden, daß sie z.B. auf einem Dopplergerät ein farbcodiertes Echo erzeugen, das dann als Markierung im Schallbild dargestellt wird.

Der Sensor 6 kann entweder direkt mit der Schallquelle z.B. über die Koppelanordnung 5 verbunden sein, wie es in Fig. 1 dargestellt ist, oder aber mittels der mechanischen Halterung 9 am Therapiekopf eines Lithotripters oder eines sonstigen Therapiegerätes mit fester Zuordnung zu dessen Fokus verbunden sein. Schließlich ist es auch möglich, den Sensor 6 unabhängig von Schallquelle 4 bzw. Therapiekopf 7 aus der freien Hand heraus einzusetzen. In diesem Fall werden entsprechende Wegaufnehmer, z.B. für die einstellbare Verzögerung des Sensors verwendet, um damit unabhängig von der verwendeten Schallquelle die Position des Therapiefokus im Schallbild 10 zu markieren.

## Patentansprüche

1. Vorrichtung zur Ortung von Konkrementen (3) im Körper (1) eines Patienten, bestehend aus einer Schallquelle (4) zur Erzeugung eines Schallbildes (10), aus einer Koppelanordnung (5) zwischen Schallquelle (4) und Patient zur Einleitung der erzeugten Schallwellen in den Körper (1) des Patienten und aus einer Anordnung zur Erzeugung einer Markierung,
dadurch gekennzeichnet, dass
die Anordnung zur Erzeugung einer Markierung aus einem für die verwendeten Schallstrahlen transparenten Sensor (6) besteht, der derart ausgelegt ist, dass er nach dem Auftreten des von der Schallquelle (4) der Ortungsanordnung ausgesandten Sendepulses auf der Fläche des Sensors (6) und nach Ablauf der einstellbaren Verzögerungszeit, ein Signal erzeugt, das als Markierung an einer der einstellbaren Verzögerungszeit entsprechenden beliebig wählbaren Stelle in das erzeugte Schallbild (10) eingeblendet wird.

2. Vorrichtung nach Anspruch 1.
dadurch gekennzeichnet, dass
die einstellbare Verzögerungszeit der doppelten Laufzeit des Schallimpulses zwischen Sensor (6) und vorgegebener Tiefe im Körper entspricht.

3. Vorrichtung nach einem der vorhergehenden Ansprüche,
dadurch gekennzeichnet, dass
die Sensorfläche im wesentlichen der Querschnittsfläche des Schallwellensenders (4) entspricht.

4. Vorrichtung nach einem der vorhergehenden Ansprüche,
dadurch gekennzeichnet, dass
der Sensor (6) aus einzelnen Zellen derart zusammengesetzt ist. dass das erzeugte Signal eine vorgegebene Form aufweist.

5. Vorrichtung nach einem der vorhergehenden Ansprüche,
dadurch gekennzeichnet, dass
der Sensor (6) direkt an der Schallquelle (4) angeordnet ist.

6. Vorrichtung nach einem der vorhergehenden Ansprüche,
dadurch gekennzeichnet, dass
der Sensor (6) über eine mechanische Halterung (9) am Therapiekopf (7) befestigt ist.

## Claims

1. Device for location of concrements (3) in the body (1) of a patient. Device consisting of sound source (4), an arrangement of coupling devices (5) between a sound source (4) and a patient for introducing the generated sound waves in the patient's body (1) and an arrangement of devices for generating a marking (6),
characterized in that
the arrangement of devices for generating a marking (6) consists of a sensor (6) transparent for the applicated sound radiation and which has been designed the way that it generates a signal when the adjustable delay time has expired and when the transmitter pulse sent by the sound source (4) of the locating device occurs within the sensor area (6). The generated signal will pop up as a marking (11) at a any place to be defined within the generated sound image (10).

2. Device according to Claim 1,
characterized in that
the adjustable delay time corresponds to the double running time of the sound impulse between the sensor (6) and the predefined depth of the body.

3. Device according to one of the previous claims
characterized in that
essentially the sensor area corrresponds to the cross section area of the sound wave transmitter(4).

4. Device according to one of the previous claims
characterized in that
the sensor (6) is composed of various cells the way that the generated signal occurs in a predefined form (11).

5. Device according to one of the previous claims
characterized in that
the sensor (6) is positioned near the sound source (4).

6. Device according to one of the previous claims
characterized in that
the sensor(6) is fixed by a mechanical support(9) onto the therapy head (7).

## Revendications

1. Dispositif pour la localisation des calculs (3) dans le corps (1) d'un patient. Ledit consiste d'une source sonore (4), et d'un arrangement des dispositifs de couplage (5) placés entre une source sonore (4) et un patient et utilisé pour introduire dans le corps d'un patient des ondes sonores générées, de même d'un arrangement pour la génération d'une marque (6),
caractérisé en ce que
l'arrangement pour la génération de la marque (6) consiste d'un détecteur (6) transparent pour la radiation acoustique (6) appliquée et qui est construit de la manière qu'il génère un signal lorsque le temps de retard ajustable a expiré, et lorsque l'impulsion émetteur envoyée par la source sonore (4) du dispositif de localisation apparaît dans l'aire de détecteur (6). Le signal généré prend la forme d'une marque dans l'image ultrasonique générée (11) et est placé à un endroit quelconque à definir (10).

2. Dispositif selon Demande 1,
caractérisé en ce que
le temps de retard ajustable correspond au double temps de propagation de l'impulse acoustique entre le détecteur (6) et la profondeur du corps.

3. Dispositif selon l'une des demandes précédentes,
caractérisé en ce que
en essentiel, l'aire de détecteur correspond à l'aire de la section transversale (4)

4. Dispositif selon l'une des demandes précédentes,
caractérisé en ce que
le détecteur (6) est composé des cellules variées de la manière que le signal généré apparaît dans une forme prédéfinie(11)

5. Dispositif selon l'une des demandes précédentes,
caractérisé en ce que
le détecteur (6) est placé directement à la source sonore (4).

6. Dispositif selon l'une des demandes précédentes,
caractérisé en ce que
le détecteur (6) est attaché à la tête thérapeutique (7) par un support mécanique (9).
